Europäisches Patentamt

European Patent Office

Office européen des brevets

(19)

(11) Publication number: **0 209 509 B1**

# EUROPEAN PATENT SPECIFICATION

(12)

(45) Date of publication of patent specification: **18.09.91**

(51) Int. Cl.⁵: **C09K 15/18**, A23K 1/16, C11B 5/00

(21) Application number: **86850257.6**

(22) Date of filing: **15.07.86**

(54) Anti-oxidants for use in the protection of easily oxidizable compounds, and a method for the protection of easily oxidizable compounds and application of the anti-oxidants.

(30) Priority: **17.07.85 NO 852860**

(43) Date of publication of application:
**21.01.87 Bulletin 87/04**

(45) Publication of the grant of the patent:
**18.09.91 Bulletin 91/38**

(84) Designated Contracting States:
**CH DE FR GB IT LI NL**

(56) References cited:
FR-A- 1 502 976
FR-M- 3 517
US-A- 3 031 485
US-A- 4 507 321

CHEMICAL ABSTRACTS, vol. 104, no. 15, 14th
April 1986, page 295, abstract no. 125403f,
Columbus, Ohio, US; B. TADOLINI et al.:
"Inhibition of lipid peroxidation by spermine
bound to phospholipid vesicles", & BIOG.
AMINES 1985, 3(2), 97-106

CHEMICAL ABSTRACTS, vol. 33, no. 21, 10th
November 1939, column 8799(2), Columbus,

Ohio, US; E.A. EVANS et al.: "Effect of sper-
mine on tissue oxidations", & PROC. SOC.
EXPTL. BIOL. MED. 41, 467-70(1939)

FOOD, SCIENCE & TECHNOLOGY AB-
STRACTS, abstract no.
86-05-j0086(86028684); A. ROVERSI: "Do
polyamines enhance fruit-setting in sweet
cherry?", & ANNALI DELLA FACOLTA DI AG-
RARIA, UNIVERSITA CATTOLICA DEL SACRO
CUORE, vol. 23, no. 2, pages 201-206, 11 ref.,
1983

(73) Proprietor: **Sparebanken Nord-Norge**
**Storg 65 Postboks 703**
**N-9000 Tromsoe(NO)**

(72) Inventor: **Lovaas, Erik**

**N-9014 Hapet(NO)**

(74) Representative: **Wennborg, Göte et al**
**KRANSELL & WENNBORG AB Sandhamns-**
**gatan 42**
**S-115 28 Stockholm(SE)**

## Description

The invention concerns anti-oxidants to be used for the protection of easily oxidizable compounds, especially polyunsaturated fatty acids, against oxidation. The anti-oxidants also have a growth-stimulating effect on fish, and may be used as an additive in feed.

Polyunsaturated marine fatty acids have attracted medical and nutritional attention in recent years because of their ability to protect against heart disease and cancer. It is supposed that the high incidence of these diseases is largely due to defective nutrition, and that the risk may be reduced by a change of eating habits or otherwise by consuming enough of the nutritionally important polyunsaturated fatty acids.

Polyunsaturated fatty acids and anti-oxidants are also of importance in the raising of domestic animals and in breeding. Fat is the cheapest source of energy in feed products, hence attempts are made to find diets containing a maximum of fats. Increasing the amount of fat does, however, increases the risk of rancidity. A balance must be found between the amount of fat it is desirable to add and the stability of the fat. In fish feed ethoxyquin is presently in use as an anti-oxidant. The compound is very effective, but is not allowed in foods for human consumption It is worrying for Norwegian fish farms that ethoxyquin has been traced in edible fish. Alternatives to ethoxyquin are not yet available.

Anti-oxidants also prevent disease. For example, Norwegian fish farms lost between 100 and 200 million kroner in 1984, as a result of infection in farmed fish. It is supposed that the diseases are linked to poor feed quality, especially rancid fat.

The present invention takes as its starting point the marked reduction in the stability of fatty acids in the presence of acid. Acid is produced when fat turns rancid, which gives a positive feed-back leading to an acceleration of the rancidity process. I have postulated that there exists a type of anti-oxidant which counteracts said process by neutralizing the acid. In order to fulfil their intended function, such anti-oxidants must have good fat-soluble properties, and they must be alkaline. Moreover, I have tried to find natural compounds of a known biochemistry.

Attention has been centred on polyamines. Said compounds occur in all living organisms, and have a simple structure of primary and secondary amino-groups which can neutralize acid. Polyamines also have physical and chemical properties that render them useful for a number of product types: they are fat and water-soluble, they flow easily, they have no marked taste or smell, and they are colourless.

The chemical structure of two natural polyamines is shown below:

SPERMIDINE $\quad$ $NH_2\text{-}CH_2\text{-}CH_2\text{-}CH_2\text{-}NH\text{-}CH_2\text{-}CH_2\text{-}CH_2\text{-}CH_2\text{-}NH_2$

SPERMINE $\quad$ $NH_2\text{-}CH_2\text{-}CH_2\text{-}CH_2\text{-}NH\text{-}CH_2\text{-}CH_2\text{-}CH_2\text{-}CH_2\text{-}NH\text{-}CH_2\text{-}CH_2\text{-}CH_2\text{-}NH_2$

Thus, the invention concerns anti-oxidants which are to be used for protection of easily oxidizable compounds, especially polyunsaturated fatty acids, against oxidization, and which are naturally occurring compounds soluble in organic as well as acqueous solvents, and which are preferably colourless and have no fluorescence and only a faint smell and taste, said compounds being characterized in that they are spermine or spermidine.

Furthermore, the invention concerns a method for the protection of easily oxidizable compounds, especially polyunsaturated fatty acids, against oxidization, the method being characterized in that the compounds are mixed with the above anti-oxidants.

The above anti-oxidants can be used for oxidation protection of marine oils such as cod liver oil, capelin oil, and their concentrates, foodstuffs such as margarine, chocolate, dressings, meat and fish, feed products such as fish feed, feed for fur-bearing animals, poultry and domestic animals, cat and dog food, cosmetics such as lipstick, and skin creams, technical products such as lubricating oils, petrol, rubber and plastic, and pharmaceuticals.

The effect of different polyamines has been tested. Table 1 shows the effect of some common anti-oxidants on capelin oil (from Mallotus villosus) containing 7% easily oxidizable polyunsaturated fatty acids (20:5, 22:6). The stability was investigated by means of an accelerated test, in which the oil was exposed to oxygen at a high temperature (100°C). The induction time, being the time from the start of the experiment until a rapid increase in a rancidity parameter takes place, is a measure of the effect of an added anti-oxidant. The effects of different anti-oxidants given as % increase in induction time (protection factor) are shown in Table 1.

Table 1

| Anti-oxidant 2mM | Protection factor |
|---|---|
| Blank | 0.0 |
| Vitamin E | 133.3 |
| BHA | 142.9 |
| Ascorbyl palmitate | 723.8 |
| Ethoxyquin | 1090.5 |
| Spermidine | 2947.6 |
| Spermine | 3957.1 |

The anti-oxidative effect of 200 ppm polyamines on a 30% concentrate of 20:5/22:6 is shown in Table 2. The peroxide development was monitored over a 60-day period. The mean temperature was 26°C.

The analyses were grouped in 7 series (A,B,C,D,E,F and G).

Series A

Samples were stored in a fridge (4°C), and samples were drawn from an unopened bottle once a week.

Series B

Samples were stored in the dark and at room temperature, and the material for analysis was taken from an unopened bottle once a week.

Series C

1 sample was stored in the dark in an open bottle at room temperature. Samples were taken 3 times a week.

The series D, E, F and G

The samples were stored in the dark in a sealed bottle and at room temperature. After sample taking, the bottles were agitated 5 times and sealed. Series D has no anti-oxidant, series E contains 200 ppm TBHQ (tertiary butyl hydroxy kinon), to series F has been added 200 ppm octyl gallate. Series G contains 200 ppm spermine. The series C and D differ from each other in that C was open constantly, whereas D was opened 3 times a week and agitated. Measuring was performed 3 times a week.

Table 2

| Series | Temp. | Conditions Open/sealed | Anti-oxidant | Oxidation rate |
|---|---|---|---|---|
| A | 4 | SEALED | | -0.027 |
| B | 26 | SEALED | | -0.177 |
| C | 26 | OPEN | | 5.397 |
| D | 26 | OPEN/SEALED | | 3.597 |
| E | 26 | OPEN/SEALED | TBHQ | 3.414 |
| F | 26 | OPEN/SEALED | OCTYL GALLATE | 3.210 |
| G | 26 | OPEN/SEALED | X | 0.090 |

The investigation shows that spermine in open bottles gives the same protection as storage in sealed bottles. TBHQ and octyl gallate do not induce significant increases in stability.

The anti-oxidative effect depends both on the type of polyamine and on its concentration (Table 3). On a molar basis, the effect of putrescine is not as favourable as that of spermine. The variation in effect corresponds closely to the number of amino groups in the various polyamines the tests were performed on 30% concentrate of 20:5/22:6. Oxygen exposure at 70°C was used.

3

Table 3.

A = spermine

B = spermidine

C = putrescine

---

### Induction Time (hours)

| mM | A | B | C |
|---|---|---|---|
| 0.0 | 369 | 351 | 354 |
| 0..1 | 501 | 393 | 393 |
| 0.5 | 1059 | 741 | 493 |
| 1.0 | 1695 | 1158 | 663 |
| 2.0 | 3156 | 2031 | 984 |

---

The anti-oxidative effect of polyamines has been confirmed through examining the fatty acid profile at different times during the oxidation process. Table 4 shows the fatty acid profile of capelin oil, stabilized by 0.8 mM spermine, at 0, 24.5 and 28.5 hours. The oil was oxidized at 100°C, and had an induction time of 24.5 hours.

Table 4

| Fatty acid | Composition after no. of hours | | | % variation from t = 0 | |
|---|---|---|---|---|---|
| | 0 | 24.5 | 28.5 | t = 24.5 | t = 28.5 |
| 18:4 | 1.6 | 1.5 | 0.7 | -6.3 | -56.3 |
| 20:5 | 4.0 | 4.0 | 1.3 | 0.0 | -67.5 |
| 22:6 | 2.1 | 2.1 | 0.5 | 0.0 | -76.2 |

Stabilisation of polyunsaturated fatty acids in fish feed is regarded as a key to increased growth, good general condition and reduced mortality.

The effect of spermine on char spawn is described below and in Table 5.

The results are:

1. 200 ppm spermine in fish feed has no apparent negative effects on the fish.

2. Mortality is reduced from 4.5% in the monitor group to 1.1% in the pilot group.

3. The growth rate increases by 20% in the pilot group (Table 5).

It is concluded that polyamines may be used in fish feed.

The growth test was performed on 4 groups of char spawn. The total number of individuals was 332. The spawn was acclimatised over a 14-day period, during which time the temperature was increased gradually from 2.5 to 8.8°C. During the acclimatisation period the spawn was fed EWOS ST40-2.

The feed given to each of the groups was:

A. Commercial EWOS ST40-2, stabilised with 200 ppm ethoxyquin.

B. Freeze-dried capelin roe with added vitamins and minerals, but with no anti-oxidant.

C. Freeze-dried capelin roe with added vitamins, minerals and 200 ppm ethoxyquin.

D. Freeze-dried capelin roe with added vitamins, minerals and 200 ppm spermine.

Table 5

| Day | 0 | 14 | 28 | 42 | 56 |
|---|---|---|---|---|---|
| Group | | Wet weight (g) | | | |
| A | 0.980 | 1.897 | 2.468 | 2.761 | 3.016 |
| B | 0.980 | 1.726 | 2.025 | 2.083 | 2.285 |
| C | 0.980 | 1.702 | 2.068 | 2.111 | 2.307 |
| D | 0.980 | 1.593 | 2.115 | 2.205 | 2.804 |
| | | % growth above monitor group (B) | | | |
| B | 0.0 | 0.0 | 0.0 | 0.0 | 0.0 |
| C | 0.0 | -1.4 | 2.1 | 1.3 | 1.0 |
| D | 0.0 | -7.7 | 4.4 | 5.9 | 22.7 |

Example 1

Marine oils, such as capelin oil, cod liver oil, cod liver oil capsules and concentrates of polyunsaturated fatty acids are stabilised by adding the polyamines at a suitable stage of the production process. In the case of oils produced at low temperatures, the anti-oxidants may be added before boiling. The concentration of the additive will depend on (a) what improvement in stabilisation is desired, and (b) what is the upper limit for anti-oxidants approved by the Health authorities.

Example 2

Foodstuffs such as margarine, chocolate and dressings are stabilised by adding the polyamines at a suitable stage of the production process. The antioxidants may be added either as an acqueous solution or mixed with oil.

Example 3

Feed products such as fish feed and feed for fur-bearing animals are stabilised by adding the anti-oxidant at a suitable stage of the production process. The polyamines may be added to the raw materials (fish, fish offal, meat offal etc.) together with other water or fat-soluble additives.

Example 4

Cosmetics (both water and fat-based) may be stabilised by mixing in the polyamines at a suitable stage of the production process.

Example 5

Technological products such as petrol, lubricating oils, plastic, rubber and floating crystals are stabilised by mixing in the anti-oxidant at a suitable stage of the production process. In the case of polymer products, this may be before initiation is carried out.

Example 6

Pharmaceuticals are stabilised by adding the polyamines in a concentration as stipulated by the Health Authorities. Polyamines may also be produced as separate pharmaceuticals and in suitable dispensary forms (tablets, retard tablets, solublets, pills, capsules, powders, granulates, mixtures, suppositories, injectabilia, infusion fluids, ampulles, dialytica, ointments, liniments, spray fluids etc.)

## Claims

1. Anti-oxidants which are to be used for protection of easily oxidizable compounds, especially polyunsaturated fatty acids, against oxidation, and which are naturally occurring compounds soluble in organic as well as acqueous solvents, and which are preferably colourless and have no fluorescence, and which have only faint smell and taste, **characterized in** that they are spermine or spermidine.

2. Anti-oxidants according to Claim 1, **characterized in** that they are growth factors for fish.

3. A method for the protection of easily oxidizable compounds, especially polyunsaturated fatty acids, against oxidization, **characterized in** that the compounds are mixed with antioxidants according to Claim 1.

4. A method for increasing the growth rate in farmed fish, **characterized in** that anti-oxidants according to Claim 1 are mixed into the feed.

5. A method according to Claims 3 and 4, **characterized in** that the anti-oxidants are used in concentrations of 2-1,000 ppm.

## Revendications

1. Anti-oxydants qui sont destinés à être utilisés pour la protection contre l'oxydation de composés facilement oxydables notamment d'acides gras polyinsaturés, et qui sont des composés naturels solubles dans des solvants organiques et également dans les solvants aqueux, et sont de préférence incolores et ne présentent pas de fluorescence et seulement une faible odeur et un faible goût, lesdits composés étant caractérisés en ce qu'il s'agit de la spermine ou de la spermidine.

2. Anti-oxydants selon la revendication 1, caractérisés en ce que ce sont des facteurs de croissance pour poissons.

3. Procédé pour la protection contre l'oxydation de composés facilement oxydables, notamment d'acides gras polyinsaturés, caractérisé en ce que les composés sont mélangés avec des anti-oxydants selon la revendication 1.

4. Procédé pour augmenter le taux de croissance du poisson d'élevage, caractérisé en ce que des anti-oxydants selon la revendication 1 sont incorporés à la nourriture.

5. Procédé selon les revendications 3 et 4, caractérisé en ce que les anti-oxydants sont utilisés à des concentrations de 2 à 1000 ppm.

## Patentansprüche

1. Antioxidantien zum Schützen Von leicht oxidierbaren Verbindungen, speziell von polyungesättigten Fettsäuren, vor Oxydation, welche Antioxidantien natürlich vorkommende, sowohl in organischen wie auch in wässerigen Lösungsmitteln lösliche, bevorzugterweise farblose und nicht fluoreszierende sowie höchstens leicht riechende und schmeckende Verbindungen sind, dadurch gekennzeichnet, dass sie Spermine oder Spermidine sind.

2. Antioxidantien gemäss Patentanspruch 1, dadurch gekennzeichnet, dass sie Wachstumsfaktoren für Fische sind.

3. Verfahren zum Schützen von leicht oxidierbaren Verbindungen, speziell von polyungesättigten Fettsäuren, vor Oxydation, dadurch gekennzeichnet, dass die Verbindungen mit Antioxidantien gemäss Patentanspruch 1 gemischt werden.

4. Verfahren zur Erhöhung der Wachstumsrate bei gezüchteten Fischen, dadurch gekennzeichnet, dass Antioxidantien gemäss dem Patentanspruch 1 dem Futter zugemischt werden.

5. Verfahren gemäss den Patentansprüchen 3 und 4, dadurch gekennzeichnet, dass die Antioxidantien in Konzentrationen von 2 bis 1000 ppm verwendet werden.